# EUROPEAN PATENT APPLICATION

(11) **EP 3 967 302 A1**
(43) Date of publication of application: **16.03.2022**
(21) Application number: 21167736.4
(22) Date of filing: 09.04.2021
(51) Int. Cl.: A61K 31/165, A61K 31/4985, A61P 1/16, A61K 31/045, A61K 31/403, A61K 31/4375, A61K 31/495, A61K 31/513, A61K 31/519, A61K 31/69, A61K 31/355, A61K 31/375, A61K 31/51

(54) **COMPOSITION FOR PREVENTING OR TREATING LIVER DISEASES**

(30) Priority: 15.09.2020 KR 20200118390
(71) Applicant: Acebiomed, Inc., Jinju, Gyeongnam 52839 (KR)
(72) Inventor: CHA, WangJo, Gwacheon-si, Gyeonggi-do (KR); YOUN, Ju Ho, Seoul (KR); HAM, Sun Ah, Chanwong-si, Gyeongsangnam-do (KR)
(74) Representative: Prüfer & Partner mbB Patentanwälte · Rechtsanwälte

(57) **Abstract**

The present invention relates to a composition for preventing or treating liver diseases, and more particularly, provides a pharmaceutical composition for preventing or treating liver diseases containing colchicine and a dipeptidyl peptidase-4 (DPP-4) inhibitor as active ingredients. The pharmaceutical composition according to the present invention may significantly reduce liver injury such as fibrosis, fat accumulation, and inflammation in hepatocytes or liver tissues and more effectively prevent or treat liver diseases by co-using colchicine and a DPP-4 inhibitor, which are drugs used for treating other diseases in the related art.

## Description

### [Technical Field]

The present invention relates to a composition for preventing or treating liver diseases, and more particularly, to a pharmaceutical composition for preventing or treating liver diseases containing colchicine and a dipeptidyl peptidase-4 (DPP-4) inhibitor as active ingredients.

### [Background Art]

The liver plays a pivotal role in the metabolism of substances introduced from the outside of the body and in the body, and is a biological organ in which enzyme reaction and energy metabolism occur continuously. Currently, among chronic diseases in Korea, hepatitis, liver cirrhosis, and liver cancer account for the highest proportions together with circulatory system diseases, and account for a large proportion of the causes of death from diseases. In particular, as the drinking population is relatively high compared to advanced countries and the cause of liver injury caused by drinking binge is high, interest in these diseases is also great.

Among the liver diseases, nonalcoholic steatohepatitis (NASH) (or nonalcoholic fatty liver disease (NAFLD)) is a disease caused by obesity, diabetes, hyperlipidemia, drugs, etc., other than drinking. As fat accumulates in hepatocytes, hepatocellular degeneration/necrosis occurs and develops into liver cirrhosis and liver cancer due to inflammation and liver fibrosis, which is recognized as a serious disease worldwide.

In particular, liver fibrosis refers to a state in which injured liver tissue is transformed into fibrous tissue such as collagen, rather than being restored to normal hepatocytes, as part of a bio-adaptive response accompanying chronic liver diseases such as hepatitis. The liver fibrosis is a bio-adaptive response that occurs in a process of repairing tissue injury, but deterioration in liver function inevitably occurs in that the liver is replaced with fibrous tissue that cannot perform its own functions at all, such as metabolism of substances in the body, secretion of bile, etc. When the liver fibrosis is continuously repeated, the liver fibrosis develops into liver cirrhosis and leads to death, and as a result, the development of an appropriate therapeutic agent has been performed as an important project in drug development. However, until now, since the mechanism itself of liver fibrosis has not been clearly found, a suitable therapeutic drug has not been developed.

As described above, since persistent injury of liver tissues has the characteristics of diseases that develop into liver cirrhosis or liver cancer, treatment and prevention of liver diseases is very important by considering the physiological characteristics and importance of liver tissue, and there is a need to develop a therapeutic agent for liver diseases that may reduce liver tissue injury and ultimately improve a therapeutic effect.

Meanwhile, colchicine has already been widely used as a therapeutic agent for gout-related arthritis, and therapeutic cases of acute gout and recurrent gout have been reported. In addition, the pharmacokinetic properties of colchicine have also been reported and the colchicines may be administered orally. For more than 10 years, the colchicine has been reported on cases of various indications and studied on mechanism, and has been reported to be effective in rheumatic and non-rheumatic arthritis, prevention of amyloidosis in familial Mediterranean fever, fever prevention, Behcet's disease, etc.

In addition, a dipeptidyl peptidase-4 (DPP-4) inhibitor is known as an anti-diabetic therapeutic agent which selectively inhibits DPP-4, which blocks the action of glucagon like peptide-1 (GLP-1) that regulates blood sugar to improve the regulation of levels of blood sugar in Type 2 diabetic patients. Although many studies and prior arts related to the treatment of liver diseases using the DPP-4 inhibitor have been reported, the effect of improving liver fibrosis is insufficient, so that there is a limit to administration for treating the disease and there have been no report on techniques for treating liver diseases using these drugs in combination so far.

### [Prior Arts]

(Patent Document) Korean Patent Publication No. 10-2009-0059017 (published on June 10, 2009)

### [Disclosure]

### [Technical Problem]

An object of the present invention is to provide a pharmaceutical composition including a co-formulation having an excellent therapeutic effect on liver diseases.

### [Technical Solution]

In order to achieve the object, the present invention provides a pharmaceutical composition for preventing or treating liver diseases containing colchicine and a dipeptidyl peptidase-4 (DPP-4) inhibitor as active ingredients. Further, the present invention provides a pharmaceutical composition for co-administration for preventing or treating liver diseases containing colchicine and a dipeptidyl peptidase-4 (DPP-4) inhibitor as active ingredients.

### [Advantageous Effects]

The present invention relates to a new combination therapy of colchicine and a DPP-4 inhibitor, drugs which have been used for treating other diseases in the related art, and the two agents are used in combination to significantly reduce liver injury such as fibrosis, fat accumulation, inflammation, etc. in hepatocytes or liver tissues.

The pharmaceutical composition according to the present invention contains the colchicine and the DPP-4 inhibitor in specific amounts to exhibit a more improved effect of preventing or treating liver diseases than in the related art, thereby more effectively preventing or treating liver diseases.

### [Description of Drawings]

FIG. 1 illustrates a result of observing a change in expression of collagen I in hepatocytes of fibrosis-induced mice according to Experimental Example of the present invention.
FIG. 2 illustrates a result of observing a change in expression of SREBP1 in hepatocytes of fat accumulation-induced mice according to Experimental Example of the present invention.
FIG. 3 illustrates a result of observing a change in expression of IL-1β in hepatocytes of inflammation-induced mice according to Experimental Example of the present invention.
FIG. 4 illustrates a result of anatomically observing changes in liver tissues in a nonalcoholic steatohepatitis (NASH)-induced model mouse according to Experimental Example of the present invention through hematoxylin and eosin (H&E) staining.
FIG. 5 illustrates a result of observing changes in hydroxyproline in liver tissues in a NASH-induced model mouse according to Experimental Example of the present invention.
FIG. 6 illustrates a result of observing changes in expression of fibrosis-related genes in liver tissues in a NASH-induced model mouse according to Experimental Example of the present invention.
FIG. 7 illustrates a result of observing changes in expression of inflammation-related genes in liver tissues in a NASH-induced model mouse according to Experimental Example of the present invention.
FIG. 8 illustrates a result of observing changes in expression of fat accumulation-related genes in liver tissues in a NASH-induced model mouse according to Experimental Example of the present invention.
FIG. 9 illustrates a result of observing changes in generation of superoxide in liver tissues in a NASH-induced model mouse according to Experimental Example of the present invention.

### [Modes for the Invention]

Hereinafter, the present invention will be described in detail.

The present inventors found that when co-administering colchicine as a gout drug and a DPP-4 inhibitor as a hypoglycemic agent, there was an effect of inhibiting liver injury such as fibrosis, fat accumulation, inflammation, and the like of hepatocytes or liver tissues in a nonalcoholic steatohepatitis (NASH) animal model induced by a high fat diet, and then completed the present invention.

The present invention provides a pharmaceutical composition for preventing or treating liver diseases containing colchicine and a dipeptidyl peptidase-4 (DPP-4) inhibitor as active ingredients.

In this specification, "colchicine" is an alkaloid component contained in seeds or bulbs of a lily family plant, Colchicum autumnale, and is represented by Chemical Formula 1 below, and is a drug for oral administration that has been widely used as a therapeutic agent for gout-related arthritis as described above.

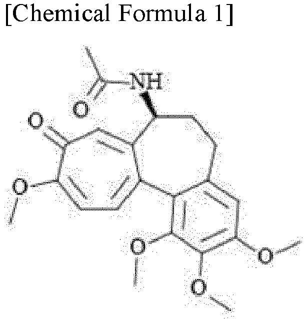

In this specification, the "dipeptidyl peptidase-4 (DPP-4) inhibitor", as described above, selectively inhibits DPP-4, which blocks the action of GLP-1 that regulates blood sugar, and in detail, may inhibit the expression of a DPP-4 nucleotide or the expression or activity of a DPP-4 protein.

In the present invention, the inhibiting of the expression of the DPP-4 nucleotide may be one or two or more selected from the group consisting of an antisense nucleotide against mRNA of DPP-4, an aptamer, small interfering RNA (siRNA), short hairpin RNA (shRNA), micro RNA (miRNA) and interference (RNAi), but is not limited thereto.

The inhibiting of the expression or activity of the DPP-4 protein may be an antibody, a peptide, a peptide mimetics, an aptamer, a compound or a natural product that specifically binds to DPP-4. The compound may be one or two or more selected from the group consisting of sitagliptin, alogliptin, anagliptin, dutogliptin, evogliptin, gosogliptin, gemigliptin, linagliptin, omarigliptin, saxagliptin, teneligliptin, trelagliptin, vildagliptin, berberine, diprotein, and lupeol, preferably sitagliptin, alogliptin, evogliptin, gemigliptin, linagliptin, saxagliptin, teneligliptin, or vildagliptin, and more preferably sitagliptin, but is not limited thereto.

The colchicine or DPP-4 inhibitor compound may be used in the form of a pharmaceutically acceptable salt within a range having the same efficacy.

As used herein, "pharmaceutically acceptable" refers to a salt having a safety and efficacy profile suitable for administration to humans without toxicity to cells or humans exposed to the composition.

The salt may be used in the form of either a pharmaceutically acceptable basic salt or acid salt. The basic salt may be used in the form of either an organic base salt or an inorganic base salt, and may be selected from the group consisting of a sodium salt, a potassium salt, a calcium salt, a lithium salt, a magnesium salt, a cesium salt, an aminium salt, an ammonium salt, a triethylaminium salt and a pyridinium salt.

The acidic salt is usefully acid addition salts formed by free acids. As the free acids, inorganic acids and organic acids may be used, and as the inorganic acids, hydrochloric acid, bromic acid, sulfuric acid, sulfurous acid, phosphoric acid, double phosphoric acid, nitric acid, etc. may be used. In addition, the organic acids may include citric acid, acetic acid, maleic acid, malic acid, fumaric acid, glucoic acid, methanesulfonic acid, benzenesulfonic acid, camphorsulfonic acid, oxalic acid, malonic acid, glutaric acid, acetic acid, glycolic acid, succinic acid, tartaric acid, 4-toluenesulfonic acid, galacturonic acid, embonic acid, glutamic acid, citric acid, aspartic acid, stearic acid, etc. However, the free acids are not limited thereto, and salts formed using various inorganic and organic acids commonly used in the art may be included.

In addition, the compound may include all salts, hydrates, solvates, derivatives, and the like that may be prepared by a conventional method, as well as the salts. The addition salts may be prepared by a general method and may be dissolved in a water-miscible organic solvent, such as acetone, methanol, ethanol, or acetonitrile and added with an excessive amount of organic base or an aqueous base solution of an inorganic base to be prepared by precipitation or crystallization. Alternatively, the addition salts may be obtained by evaporating and drying a solvent or excess base from the mixture or prepared by suctioning and filtering the precipitated salt.

In the present invention, the composition may contain colchicine and a DPP-4 inhibitor in a weight ratio of 1:(100 to 10000), preferably 1:(100 to 6000), more preferably 1:(200 to 3000), but is not limited thereto.

For example, in the case of sitagliptin, the composition may contain colchicine and sitagliptin in a weight ratio of 1:(100 to 3000), preferably 1:(250 to 2000), more preferably 1:(500 to 1500), but is not limited thereto.

In the present invention, the composition may contain 0.01 to 300 µg/kg of the colchicine and 0.01 to 300 mg/kg of the DPP-4 inhibitor, preferably 10 to 100 µg/kg of the colchicine and 5 to 120 mg/kg of the DPP-4 inhibitor, but is not limited thereto.

For example, in the case of sitagliptin, the composition may contain 10 to 100 µg/kg of the colchicine and 5 to 120 mg/kg of the sitagliptin, preferably 20 to 80 µg/kg of the colchicine and 10 to 80 mg/kg of the sitagliptin, more preferably 30 to 50 µg/kg of the colchicine and 20 to 50 mg/kg of the sitagliptin, but is not limited thereto.

When the content of the colchicine or DPP-4 inhibitor is less than the above range, its efficacy is hardly exhibited, and when the content thereof exceeds the above range, side effects such as gastrointestinal disorders may occur at the time of administration, so that the above range is preferable.

In particular, according to Experimental Example of the present invention, when the colchicine is 40 µg/kg and the sitagliptin is 20 mg/kg, it can be seen that there is the most significant effect on inhibition of liver fibrosis, fat accumulation or inflammation.

In the present invention, the composition may inhibit fibrosis, fat accumulation or inflammation of hepatocytes or liver tissues.

According to an Experimental Example of the present invention, the composition significantly reduced the expression of collagen I induced by transforming growth factor beta 1 (TGF-β1) and significantly reduced the expression of sterol regulatory element-binding transcription factor 1 (SREBP1) or interleukin 1 beta (IL-1β) induced by palmitate in hepatocytes AML12 of mice.

In particular, when the colchicine and sitagliptin among the DPP-4 inhibitors were co-treated, according to an Experimental Example of the present invention, it can be seen that the composition significantly reduced the expression of collagen I induced by TGF-β1 in hepatocytes AML12 of mice than that in co-treatment with other DPP-4 inhibitors. In an NASH mouse model induced by a high fat diet, it can be seen that the expression of fibrosis-related factors TGF-β1, collagen I, and α-smooth muscle actin (α-SMA) was significantly reduced, and the accumulation of hydroxyproline, an indicator of collagen, is also significantly inhibited.

In addition, it can be seen that the expression of SREBP1 and IL-β1 induced by palmitate in hepatocytes AML12 of mice was significantly reduced compared to a case of co-treatment with other DPP-4 inhibitors, and in the NASH mouse model induced by a high fat diet, the expression of fat accumulation-related factors SREBP1, diglyceride acyltransferase (Dgat), and apolipoprotein A1 (ApoA1) and levels of cholesterol and triglyceride were also significantly reduced. It can be seen that the expression of the inflammation-related factors TNF-α (tumor necrosis factor-α), IL-1β, and IL-6 (interleukin 6) is also significantly reduced.

In the present invention, the composition may reduce one or two or more hepatocyte injury factors selected from the group consisting of bilirubin, alanine aminotransferase (ALT), and aspartate aminotransferase (AST).

In addition, the composition may reduce the production of reactive oxygen species (ROS).

Since the composition has such an effect, the composition may be used as a pharmaceutical composition for preventing or treating liver diseases. More details will be described below by the following Experimental Examples.

In the present invention, the liver disease may be one or two or more diseases selected from the group consisting of liver fibrosis, liver cirrhosis, liver cancer, and inflammatory liver disease, but is not limited thereto, and may include all related diseases well-known in the art.

In this specification, "inflammatory liver disease" is generally referred to as hepatitis, and refers to an overall disease caused by inflammation of hepatocytes and liver tissues.

The inflammatory liver disease may be one or two or more selected from the group consisting of hepatitis, acute hepatitis, chronic hepatitis, alcoholic hepatitis, nonalcoholic steatohepatitis, subacute hepatitis, viral hepatitis, toxic liver disease, liver abscess, granulomatous hepatitis, autoimmune hepatitis, and lupus-shaped hepatitis, more preferably nonalcoholic steatohepatitis, but is not limited thereto and may include all related diseases well-known in the art.

Further, the present invention provides a pharmaceutical composition for co-administration for preventing or treating liver diseases containing colchicine and a dipeptidyl peptidase-4 (DPP-4) inhibitor as active ingredients.

According to an Experimental Example of the present invention, when the colchicine and the DPP-4 inhibitor are co-treated, liver injury such as fibrosis, fat accumulation, inflammation, etc. of hepatocytes or liver tissues was more significantly inhibited to more enhance an effect of preventing or treating liver diseases.

Features corresponding thereto may be substituted in the above-described part.

The pharmaceutical composition according to the present invention may be prepared according to a general method in a pharmaceutical field. The pharmaceutical composition may be mixed with an appropriate pharmaceutically acceptable carrier according to a formulation, and if necessary, may be prepared by further including excipients, diluents, dispersants, emulsifiers, buffers, stabilizers, binders, disintegrants, solvents, etc. The appropriate carrier or the like does not inhibit the activity and properties of the colchicine or the DPP-4 inhibitor according to the present invention, or a pharmaceutically acceptable salt thereof, and may be selected differently depending on a dosage form and a formulation.

The carrier, the excipient, the diluent, and the like that may be included in the pharmaceutical composition may include lactose, dextrose, sucrose, sorbitol, mannitol, xylitol, erythritol, maltitol, starch, acacia rubber, alginate, gelatin, calcium phosphate, calcium silicate, cellulose, methyl cellulose, microcrystalline cellulose, polyvinylpyrrolidone, water, methyl hydroxybenzoate, propyl hydroxybenzoate, talc, magnesium stearate, mineral oil, etc. When the composition is formulated, the formulation may be prepared by using diluents or excipients, such as a filler, an extender, a binder, a wetting agent, a disintegrating agent, and a surfactant which are generally used.

The pharmaceutical composition according to the present invention may be applied in any formulation, and specifically, may be formulated and used in the form of oral formulations such as powders, granules, tablets, capsules, suspensions, emulsions, syrups, aerosols, etc., external preparations, suppositories, and sterile injectable solutions according to a general method, and preferably formulated and used in a unit dosage form suitable for oral administration.

More specifically, a solid formulation of the oral formulations, in the form of tablets, pills, powders, granules, capsules, etc., may be prepared by mixing at least one excipient such as starch, calcium carbonate, sucrose, lactose, sorbitol, mannitol, cellulose, gelatin, etc., and may also include lubricants such as magnesium stearate and talc in addition to simple excipients. In addition, in the case of the capsule formulation, in addition to the above-mentioned substances, a liquid carrier such as fatty oil may be further included.

A liquid formulation of the oral formulations may correspond to suspensions, oral liquids, emulsions, syrups, and the like, and may include various excipients, for example, a wetting agent, a sweetener, an aromatic agent, a preserving agent, and the like, in addition to water and liquid paraffin which are commonly used as simple diluents.

The parenteral formulation may include sterilized aqueous solutions, non-aqueous solutions, suspensions, emulsions, injections, lyophilized preparations, suppositories, and the like. As the non-aqueous solution and the suspension, propylene glycol, polyethylene glycol, vegetable oil such as olive oil, injectable ester such as ethyl oleate, and the like may be used. As a base of the suppository, witepsol, macrogol, Tween 61, cacao butter, laurinum, glycerogelatin, and the like may be used. The present invention is not limited thereto, and all suitable preparations well-known in the art may be used.

In addition, the pharmaceutical composition according to the present invention may further add an antioxidant to enhance therapeutic efficacy. The antioxidant may use vitamin B group compounds such as thiamine (vitamin B1), riboflavin (vitamin B2), niacin (vitamin B3), pantothenic acid (vitamin B5), pyridoxine (vitamin B6), and cobalamin (vitamin B12), vitamin C, vitamin D, vitamin E, and the like, but is not limited thereto, and all suitable preparations well-known in the art may be used.

The pharmaceutical composition according to the present invention may be administered in a pharmaceutically effective dose.

The term "pharmaceutically effective dose" used herein means an amount enough to treat diseases at a reasonable benefit/risk ratio applicable to medical treatment without causing side effects.

The effective dose level of the pharmaceutical composition may be determined differently depending on factors including the purpose of use, the patient's age, sex, weight and health condition, the type of disease, the severity, the activity of a drug, the sensitivity to a drug, a method of administration, an administration time, a route of administration, a rate of excretion, a treatment period, and mixed or co-used drugs, and other factors well-known in the medical field. For example, although not constant, the pharmaceutical composition may be generally administered in an amount of 0.001 to 100 mg/kg, preferably 0.01 to 10 mg/kg once to several times a day. The dose does not limit the scope of the present invention in any aspect.

The pharmaceutical composition according to the present invention may be administered to any animal that may develop liver disease, and the animal may include, for example, not only humans and primates, but also livestock such as cattle, pigs, horses, and dogs.

The pharmaceutical composition may be administered by an appropriate route of administration according to a form of the preparation, and may be administered through various oral or parenteral routes, as long as the pharmaceutical composition may reach a target tissue. The method of administration is not particularly limited, and may be conventional methods, such as oral, rectal or intravenous, intramuscular, skin application, inhalation in the respiratory tract, intrauterine dura mater or intracerebroventricular injection.

The pharmaceutical composition according to the present invention may be used alone for the prevention or treatment of liver diseases, or may be used in combination with surgery or other drug treatment.

In addition, the present invention provides a co-administration method for preventing or treating liver diseases including treating colchicine and a dipeptidyl peptidase-4 (DPP-4) inhibitor to a subject in need of treatment for liver diseases.

The subject may be any animal other than humans.

The colchicine and the DPP-4 inhibitor may be treated simultaneously, separately or sequentially for preventing or treating liver diseases.

Features corresponding thereto may be substituted in the above-described part.

Hereinafter, the present invention will be described in detail with reference to Examples for understanding. However, the following Examples are merely illustrative of the contents of the present invention, and the scope of the present invention is not limited to the following Examples. Examples of the present invention will be provided for more completely explaining the present invention to those skilled in the art.

### <Experimental Example 1>

### <Experiment method>

### 1. Experimental materials

AML12 cells (CRL-2254) were purchased from ATCC. Colchicine (C9754), sodium palmitate (P9767), and dihydroethidium (DHE; D7008) were purchased from Sigma-Aldrich. Alogliptin (brand name: Nesina) from Korea Takeda Pharmaceuticals, evogliptin (brand name: Suganon) from Dong-A ST, gemigliptin (brand name: Zemiglo) from LG Life Science, saxagliptin (brand name: Onglyza) from Bristol-Myers Squibb, sitagliptin (brand name: Januvia) from Merck, teneligliptin (brand name: Ziten) from Glenmark Pharmaceuticals, and vildagliptin (brand name: Galvus) from Novartis were purchased.

### 2. Cell culture and drug treatment

Mouse hepatocytes, AML12 cells, were incubated at 37°C, 95% air and 5% CO₂ in a DMEM/F12 medium containing 5 µg/ml insulin, 5 µg/ml transferrin, 5 ng/ml selenium, 40 ng/ml dexamethasone, 10% fetal bovine serum (FBS) and 1% penicillin/streptomycin. AML12 cells pretreated with 5 nM of colchicine and 30 µg/ml of a dipeptidyl peptidase-4 (DPP-4) inhibitor were treated with TGF-β1 (5 ng/ml) or 0.2 mM of palmitate after 8 hours, and further incubated for additional 16 hours.

### 3. Animal testing

C57BL/6J male mice divided by 8 mice per group were stabilized for 7 days, and then a normal diet (ND) or a high fat diet (HFD) was performed for 6 weeks. Thereafter, 40 µg/kg of colchicine or 5, 10, and 20 mg/kg of sitagliptin was administered orally once a day, and a normal diet or a high fat diet was performed for additional 24 weeks. After a total of 30 weeks, after inhalation anesthesia with isoflurane, blood was obtained by using a mouse cardiac blood sampling method, and then the serum obtained by centrifugation at 3,000 rpm, 4°C for 15 minutes was used for a blood biochemical test. In addition, hydroxyproline analysis, superoxide production analysis, mRNA expression analysis, and H&E staining were performed using liver tissues.

### 4. Bilirubin assay

Bilirubin of mice was measured using a Bilirubin assay kit (Cell Biolabs). The prepared serum was dispensed into a 96 well plate by 50 µl, and 50 µl of 50% dimethyl sulfoxide (DMSO) was added. Thereafter, 125 µl of a reaction mix was added and reacted at room temperature for 30 minutes. After 75 µl of a total bilirubin probe was added, the reaction mix reacted for 20 minutes at room temperature by shielding light, and absorbance was measured at 600 nm using a microplate reader.

### 5. AST enzyme activity assay

The activity of aspartate aminotransferase (AST) of mice was measured using an AST activity assay kit (Abcam). The prepared serum was dispensed into a 96 well plate by 50 µl, added with 150 µl of a reaction reagent, and further reacted at 37°C for 60 minutes in a light-shielded state, and then absorbance was measured at 450 nm using a microplate reader.

### 6. ALT enzyme activity assay

The activity of alarnine aminotransferase (ALT) of mice was measured using an ALT activity assay kit (Sigma-Aldrich). The prepared serum was dispensed into a 96 well plate by 50 µl, added with 100 µl of a reaction reagent, and further reacted at 37°C for 60 minutes in a light-shielded state, and then absorbance was measured at 570 nm using a microplate reader.

### 7. Cholesterol assay

A total cholesterol amount of mice was measured using a Total cholesterol assay kit (Cell Biolabs). The mouse serum was diluted in a 1x assay diluent solution at a ratio of 1:100, and then dispensed into a 96 well plate by 50 µl. Thereafter, 50 µl of a cholesterol reaction reagent was added, and the reaction was performed at 37°C for 45 minutes by shielding light. For the sample after the reaction, absorbance was measured at 540 to 570 nm using a microplate reader.

### 8. Triglyceride assay

Triglyceride levels in mice were measured using an EZ-Triglyceride quantification assay kit (Dogen Bio). 1 ml of 5% NP-40 was added to a mouse serum sample, homogenized on ice, and then sufficiently cooled at room temperature. The sample in which all triglycerides were dissolved by heating once more was centrifuged at 13,000 rpm for 2 minutes, and then an insoluble material was removed, and the sample was diluted 10-fold with distilled water before assay. 2 µl of lipase was added to the sample dispensed in a 96 well plate by 90 µl and reacted for 20 minutes at room temperature, and then the reaction mixture was added to each sample by 50 µl, and further reacted at room temperature for 30 minutes in a light-shielded state. Thereafter, absorbance was measured at 570 nm using a microplate reader.

### 9. Hematoxylin and Eosin (H&E) staining method

For H&E staining, deparaffinized tissues were reacted with a 50% hematoxylin solution at room temperature for 20 minutes, reacted with 1% HCl for 1 second, and then immediately transferred to a 0.5% eosin solution and stained for 20 minutes. Thereafter, the tissue was washed with distilled water, reacted with graded alcohol and finally dehydrated with xylene. The dehydrated tissue was fixed on a coverslip using a permount.

### 10. Hydroxyproline assay

100 mg of liver tissue was homogenized with physiological saline, and then a liver tissue supernatant was separated. The separated supernatant was hydrolyzed at 120°C for 3 hours by adding the same amount of 12 N HCl. The cooled sample was vortexed and then centrifuged at 10,000 g for 3 minutes, and absorbance was measured at 550 nm using a final microplate reader by using a hydroxyproline assay kit.

### 11. Quantitative PCR method

Total RNA was extracted using an RNeasy mini kit (Qiagen), and then synthesized into cDNA using a high-capacity cDNA reverse transcription kit (Thermo Fisher). The same amount of cDNA was amplified using a SYBRTM green PCR master mix and a 10 pM primer set for each gene. The amplification process was subjected to an initial denaturation process at 95°C for 5 minutes, and repetitively replication at 95°C for 10 seconds, 58.5°C for 10 seconds, and 72°C for 10 seconds for a total of 40 times.

### 12. Superoxide assay

In order to measure production of superoxide in liver tissues of mice, dihydroethidium (DHE) was used as a superoxide fluorescent indicator. In summary, deparaffinized liver tissue slides were reacted at 37°C for 30 minutes with 10 µM DHE in a light-shielded humidity chamber. After the reaction, fluorescence indicating DHE was measured through a filter in a wavelength band of 580 nm in an Olympus FV1000 fluorescence microscope.

### <Experimental Results>

### 1. Confirmation of fibrosis inhibitory effect in hepatocytes by co-treatment of colchicine and DPP-4 inhibitor

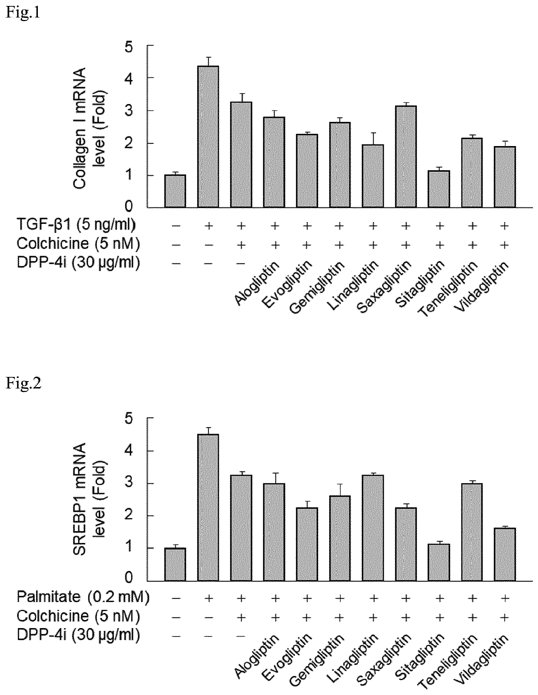

FIG. 1 illustrates a result of observing a change in the expression of collagen I in hepatocytes of fibrosis-induced mice according to an Experimental Example of the present invention. Referring to FIG. 1, it was confirmed that the expression of collagen I increased by TGF-β1 in hepatocytes AML12 of mice was reduced by treatment with colchicine alone.

Further, in order to confirm an effect of co-administration of colchicine and a DPP-4 inhibitor, as a result of reagent treatment of various DPP-4 inhibitors (alogliptin, evogliptin, gemigliptin, linagliptin, saxagliptin, sitagliptin, teneligliptin, and vildagliptin] with colchicine, it was confirmed that the DPP-4 inhibitors, excluding alogliptin and saxagliptin, exhibited a slight additive interaction in inhibiting the expression of collagen I with colchicine. In addition, it was confirmed that among them, particularly, sitagliptin had the most excellent additive interaction to inhibit fibrosis with colchicine.

### 2. Confirmation of fat-accumulation inhibitory effect in hepatocytes by co-treatment of colchicine and DPP-4 inhibitor

FIG. 2 illustrates a result of observing a change in the expression of SREBP1 in hepatocytes of fat accumulation-induced mice according to an Experimental Example of the present invention. Referring to FIG. 2, it was confirmed that the expression of SREBP1 (sterol regulatory element-binding transcription factor 1) as a fat-accumulation indicator induced by palmitate in hepatocytes AML12 of mice was reduced by treatment with colchicine alone.

Further, in order to confirm an effect of co-administration of colchicine and a DPP-4 inhibitor, as a result of reagent treatment of various DPP-4 inhibitors with colchicine, it was confirmed that the DPP-4 inhibitors, excluding linagliptin, alogliptin, and teneligliptin, exhibited a slight additive interaction in inhibiting the expression of SREBP1 with colchicine. In addition, it was confirmed that among them, particularly, sitagliptin and colchicine had the most excellent additive interaction of inhibiting the expression of SREBP1.

### 3. Confirmation of anti-inflammatory effect in hepatocytes by co-treatment of colchicine and DPP-4 inhibitor

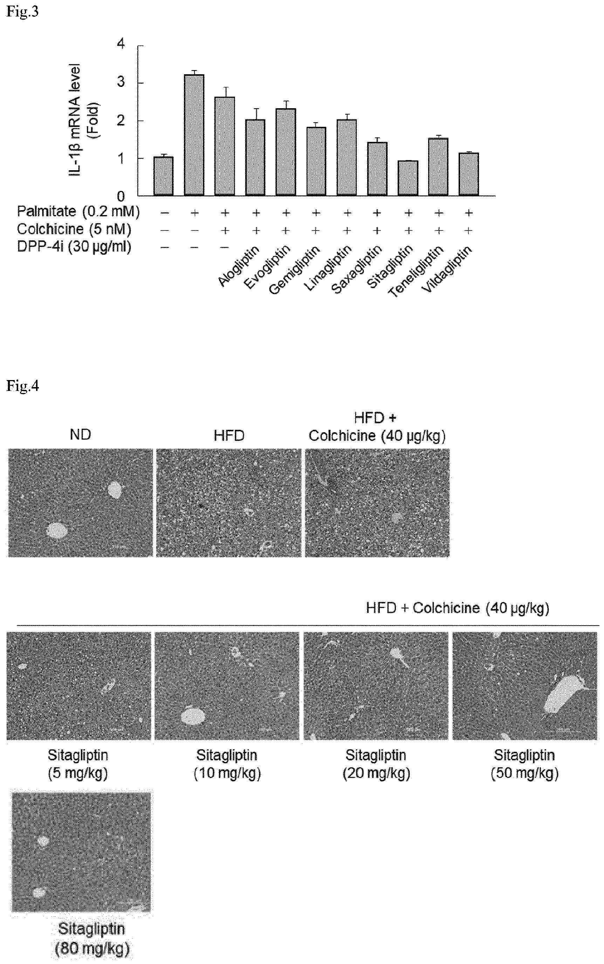

FIG. 3 illustrates a result of observing a change in the expression of IL-1β in hepatocytes of inflammation-induced mice according to an Experimental Example of the present invention. Referring to FIG. 3, it was confirmed that the expression of IL-1β (Interleukin 1 beta) as an inflammation indicator induced by palmitate in hepatocytes AML12 of mice was reduced by treatment with colchicine alone.

Further, in order to confirm an effect of co-administration of colchicine and a DPP-4 inhibitor, as a result of reagent treatment of various DPP-4 inhibitors with colchicine, it was confirmed that all the DPP-4 inhibitors exhibited a slight additive interaction in inhibiting the expression of IL-1β with colchicine. In addition, it was confirmed that among them, particularly, sitagliptin and colchicine had the most excellent additive interaction of inhibiting the expression of IL-1β.

### 4. Confirmation of fat-accumulation inhibitory effect in liver tissues by co-treatment of colchicine or sitagliptin

FIG. 4 illustrates a result of anatomically observing changes in liver tissues of mice in a nonalcoholic steatohepatitis (NASH)-induced model according to Experimental Example of the present invention through hematoxylin and eosin (H&E) staining. Referring to FIG. 4, it was confirmed that the fat accumulation in liver tissues induced by a high fat diet for 30 weeks was slightly reduced by administration of colchicine alone. In addition, it can be seen that a steatosis reduction effect of colchicine exhibits an additive interaction with sitagliptin by co-administration for each sitagliptin concentration.

### 5. Confirmation of collagen inhibitory effect in liver tissues by co-treatment of colchicine or sitagliptin

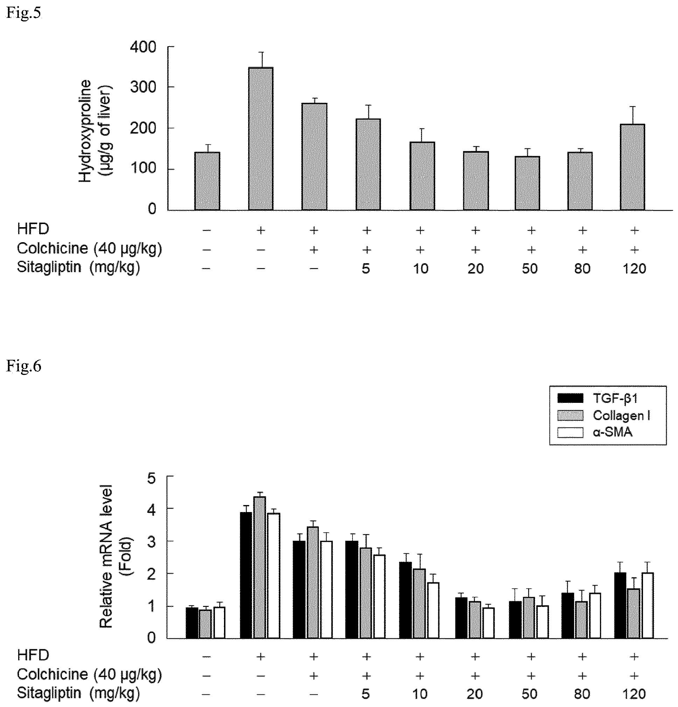

FIG. 5 illustrates a result of observing changes in hydroxyproline in liver tissues in a NASH-induced model mouse according to Experimental Example of the present invention. Referring to FIG. 5, it was confirmed that the accumulation of hydroxyproline as a collagen indicator induced by a high fat diet for 30 weeks was reduced by administration of colchicine alone. In addition, it can be seen that a hydroxyproline inhibitory effect of colchicine exhibits an additive interaction with sitagliptin by co-administration for each sitagliptin concentration.

### 6. Confirmation of fibrosis inhibitory effect in liver tissues by co-treatment of colchicine or sitagliptin

FIG. 6 illustrates a result of observing changes in expression of fibrosis-related genes in liver tissues in a NASH-induced model mouse according to Experimental Example of the present invention. Referring to FIG. 6, it was confirmed that the expression of TGF-β1, collagen I, and α-SMA (α-smooth muscle actin) as fibrosis indicator genes induced by a high fat diet for 30 weeks was reduced by administration of colchicine alone. In addition, it can be seen that an effect of reducing the expression of the fibrosis-related genes of colchicine exhibits an additive interaction with sitagliptin by co-administration for each sitagliptin concentration.

### 7. Confirmation of anti-inflammatory effect in liver tissues by co-treatment of colchicine or sitagliptin

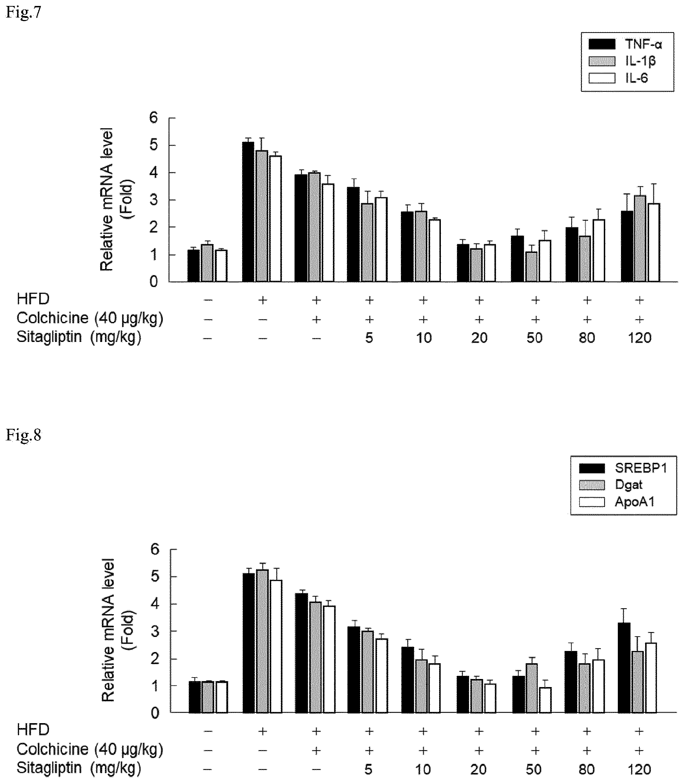

FIG. 7 illustrates a result of observing changes in expression of inflammation-related genes in liver tissues in a NASH-induced model mouse according to Experimental Example of the present invention. Referring to FIG. 7, it was confirmed that the expression of TNF-α, IL-1β, and IL-6 as inflammation indicator genes induced by a high fat diet for 30 weeks was reduced by administration of colchicine alone. In addition, it can be seen that an effect of reducing the expression of the inflammation-related genes of colchicine exhibits an additive interaction with sitagliptin by co-administration for each sitagliptin concentration.

### 8. Confirmation of fat-accumulation inhibitory effect in liver tissues by co-treatment of colchicine or sitagliptin

FIG. 8 illustrates a result of observing changes in expression of fat accumulation-related genes in liver tissues in a NASH-induced model mouse according to Experimental Example of the present invention. Referring to FIG. 8, it was confirmed that the expression of SREBP1, Dgat (Diglyceride acyltransferase), and ApoA1 (Apolipoprotein A1) as fat-accumulation indicator genes induced by a high fat diet for 30 weeks was reduced by administration of colchicine alone. In addition, it can be seen that an effect of reducing the expression of the fat accumulation-related genes of colchicine exhibits an additive interaction with sitagliptin by co-administration for each sitagliptin concentration.

### 9. Confirmation of ROS inhibitory effect in liver tissues by co-treatment of colchicine or sitagliptin

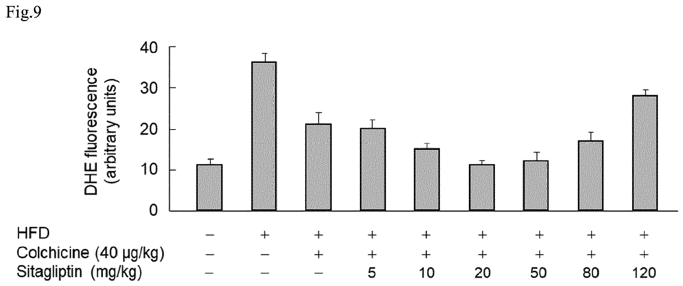

FIG. 9 illustrates a result of observing changes in generation of superoxide in liver tissues in a NASH-induced model mouse according to Experimental Example of the present invention. Referring to FIG. 9, it was confirmed that the production of superoxide as a ROS indicator induced by a high fat diet for 30 weeks was reduced by administration of colchicine alone. In addition, it can be seen that an effect of reducing the production of superoxide of colchicine exhibits an additive interaction with sitagliptin by co-administration for each sitagliptin concentration.

### 10. Confirmation of blood biochemical change by co-treatment with colchicine or sitagliptin

Table 1 shows results of performing a blood biochemical test in the serum of a NASH-induced model mouse according to an Experimental Example of the present invention. It was confirmed that a high-fat diet for 30 weeks significantly increased the levels of hepatocyte injury indicators, such as bilirubin, alanine aminotransferase (ALT), and aspartate aminotransferase (AST), and fat-accumulation indicators such as cholesterol and triglyceride in the serum of mice, but the increased levels were decreased by administration of colchicine alone. In addition, when sitagliptin was co-administered with colchicine by concentration, it is possible to observe an additive interaction that decreases further by concentration than an effect of colchicine alone.

**<Table 1>**

| **Group** | **Bilirubin (mg/dL)** | **ALT (U/L)** | **AST (U/L)** | **Total cholesterol (mg/dL)** | **Triglyceride (mg/dL)** |
|---|---|---|---|---|---|
| ND | 0.24 ± 0.05 | 54.4 ± 5.63 | 84.9 ± 1.24 | 105.5 ± 0.74 | 95.0 ± 0.50 |
| HFD | 0.71 ± 0.08 | 190.7 ± 2.25 | 245.1 ± 4.67 | 342.4 ± 0.97 | 250.6 ± 0.31 |
| HFD + Colchicine | 0.57 ± 0.05 | 154.7 ± 4.24 | 200.7 ± 2.74 | 264.8 ± 0.87 | 201.9 ± 0.27 |
| HFD + Colchicine +Sitagliptin (5 mg/ml) | 0.44 ± 0.06 | 129.3 ±4.44 | 150.3 ± 2.92 | 221.7 ± 0.77 | 173.3 ± 0.34 |
| HFD + Colchicine +Sitagliptin (10 mg/ml) | 0.36 ± 0.07 | 87.6 ± 3.90 | 124.5 ± 6.30 | 186.4 ± 0.96 | 151.5 ± 0.40 |
| HFD + Colchicine +Sitagliptin (20 mg/ml) | 0.29 ± 0.07 | 64.7 ± 3.45 | 92.7 ± 4.47 | 131.4 ± 0.94 | 114.8 ± 0.47 |
| HFD + Colchicine +Sitagliptin (50 mg/ml) | 0.30 ± 0.08 | 67.1 ± 2.21 | 103.1 ± 2.17 | 125.4 ± 0.66 | 126.4 ± 0.35 |
| HFD + Colchicine +Sitagliptin (80 mg/ml) | 0.46 ± 0.09 | 73.5 ± 2.62 | 126.7 ± 2.34 | 161.8 ± 0.52 | 156.2 ± 0.41 |
| HFD + Colchicine +Sitagliptin (120 mg/ml) | 0.58 ± 0.06 | 130.4 ± 3.15 | 172.8 ± 4.46 | 205.5 ± 0.35 | 184.8 ± 0.27 |

As described above, specific parts of the present invention have been described in detail, and it will be apparent to those skilled in the art that these specific techniques are merely preferred embodiments, and the scope of the present invention is not limited thereto. That is, the substantial scope of the present invention is defined by the appended claims and their equivalents.

## Claims

1. A pharmaceutical composition for use in treating nonalcoholic steatohepatitis comprising colchicine and a dipeptidyl peptidase-4 (DDP-4) inhibitor as active ingredients, wherein the DDP-4 inhibitor is one or two or more selected from the group consisting of sitagliptin, alogliptin, anagliptin, dutogliptin, evogliptin, gosogliptin, gemigliptin, linagliptin, omarigliptin, saxagliptin, teneligliptin, trelagliptin, vildagliptin, berberine, diprotein, and lupeol.

2. The pharmaceutical composition for use in treating nonalcoholic steatohepatitis of claim 1, wherein the composition contains the colchicine and the DPP-4 inhibitor in a weight ratio of 1:(100 to 10000).

3. The pharmaceutical composition for use in treating nonalcoholic steatohepatitis of claim 1 or 2, wherein the composition contains 0.01 to 300 µg/kg of the colchicine and 0.01 to 300 mg/kg of the DPP-4 inhibitor.

4. The pharmaceutical composition for use in treating nonalcoholic steatohepatitis of anyone of claims 1 to 3, wherein the composition inhibits fibrosis, fat accumulation, or inflammation of hepatocytes or liver tissues.

5. The pharmaceutical composition for use in treating nonalcoholic steatohepatitis of anyone of claims 1 to 4, wherein the composition reduces one or two or more hepatocyte injury factors selected from the group consisting of bilirubin, alanine aminotransferase (ALT), and aspartate aminotransferase (AST).

6. The pharmaceutical composition for use in treating nonalcoholic steatohepatitis of any one of claims 1 to 5, wherein the composition is prepared by one or two or more formulations selected from the group consisting of powders, granules, tablets, capsules, suspensions, emulsions, syrups, aerosols, external preparations, suppositories, and injectable solutions.

7. The pharmaceutical composition for use in treating nonalcoholic steatohepatitis of any one of claims 1 to 6, wherein the composition further comprises one or two or more ingredients selected from the group consisting of lactose, dextrose, sucrose, sorbitol, mannitol, xylitol, erythritol, maltitol, starch, acacia rubber, alginate, gelatin, calcium phosphate, calcium silicate, cellulose, methyl cellulose, microcrystalline cellulose, polyvinylpyrrolidone, water, methyl hydroxybenzoate, propyl hydroxybenzoate, talc, and magnesium stearate.

8. The pharmaceutical composition for use in treating nonalcoholic steatohepatitis of any one of claims 1 to 7, wherein the composition further comprises one or two or more ingredients selected from the group consisting of thiamine, riboflavin, niacin, pantophenic acid, pyridoxine, cobalamin, vitamin C, vitamin D, and vitamin E.

9. A pharmaceutical composition for co-administration for use in treating nonalcoholic steatohepatitis comprising colchicine and a dipeptidyl peptidase-4 (DDP-4) inhibitor as active ingredients, wherein the DDP-4 inhibitor is one or two or more selected from the group consisting of sitagliptin, alogliptin, anagliptin, dutogliptin, evogliptin, gosogliptin, gemigliptin, linagliptin, omarigliptin, saxagliptin, teneligliptin, trelagliptin, vildagliptin, berberine, diprotein, and lupeol.

10. The pharmaceutical composition for use in treating nonalcoholic steatohepatitis of claim 9, wherein he colchicine and the DPP-4 inhibitor are co-administered simultaneously, separately or sequentially.
